# EUROPÄISCHE PATENTSCHRIFT

(11) **EP 0 910 548 B1**
(45) Veröffentlichungstag und Bekanntmachung des Hinweises auf die Patenterteilung: **24.04.2002**
(21) Anmeldenummer: 97932771.5
(22) Anmeldetag: 03.07.1997
(51) Int. Cl.: C01B 31/30

(54) **VERFAHREN ZUR HERSTELLUNG VON ÜBERGANGSMETALLCARBIDEN SOWIE DEREN VERWENDUNG ALS KATALYSATOREN**
METHOD FOR MANUFACTURING TRANSITION-METAL CARBIDES, AND THEIR USE AS CATALYSTS
PROCEDE POUR LA PRODUCTION DE CARBURES DE METAL DE TRANSITION ET LEUR UTILISATION COMME CATALYSEURS

(30) Priorität: 12.07.1996 DE 19628160
(43) Veröffentlichungstag der Anmeldung: 28.04.1999
(73) Patentinhaber: Studiengesellschaft Kohle mbH, 45470 Mülheim (DE)
(72) Erfinder: BOGDANOVIC, Borislav, D-45470 Mülheim an der Ruhr (DE); SCHWICKARDI, Manfred, D-45470 Mülheim an der Ruhr (DE)
(74) Vertreter: von Kreisler, Alek, Dipl.-Chem.
(86) Internationale Anmeldenummer: EP9703515
(87) Internationale Veröffentlichungsnummer: WO9802383

(56) Entgegenhaltungen:
- WO-A-95/28352
- DE-A- 2 747 016
- US-A- 4 812 301

## Beschreibung

Die vorliegende Erfindung betrifft ein Verfahren zur Herstellung von neuen oder bekannten Übergangsmetall-Kohlenstoff-Verbindungen (Übergangsmetallcarbiden) sowie deren Verwendung als Katalysatoren.

Nach dem US Patent 5.385.716 (1995) bzw. der europäischen Patentanmeldung 0469463 (1991), lassen sich durch Umsetzung von Übergangsmetallchloriden mit Magnesium oder magnesiumorganischen Verbindungen in z.B. Tetrahydrofuran (THF) lösliche Übergangsmetall-Magnesiumchlorid-Komplexe der allgemeinen Formel [M(MgCl)ₘ(MgCl₂)ₙ], M = Übergangsmetall, m = 1,2,3, n = 0 - 1, die sogenannten "anorganischen Grignard-Reagenzien", darstellen.

Es wurde nun überraschenderweise gefunden, daß die anorganischen Grignard-Reagenzien mit Perchloralkanen bzw. -alkenen sowie mit Perchloraromaten, in z.B. THF, unter Eliminierung des organisch gebundenen Chlors in Form von MgCl₂ und unter gleichzeitiger Bildung von Übergangsmetallcarbiden von Metallen der 4. - 10. Gruppe des Periodensystems reagieren. Bevorzugt sind Metalle der 4. und 10. Guppe, und hier wieder Titan, Ruthenium, Iridium und Rhodium. Dieser Befund ist deshalb überraschend, weil die genannten organischen Chlorverbindungen gegenüber nucleophilen Reaktionspartnern, wie z.B. gegenüber Metallen oder metallorganischen Verbindungen, als ausgesprochen reaktionsträge gelten. Die so dargestellten, neuen oder bekannten Metallcarbide erweisen sich als hochaktive heterogene Hydrierkatalysatoren wirksam. Im folgenden wird die Erfindung anhand einiger Beispiele näher erläutert.

Die gemäß der zitierten Literatur darstellbaren Grignard-Reagenzien des Rutheniums und des Iridiums, [Ru(MgCl)₃] und Ir(MgCl)₃], reagieren mit Tetrachlorethen im Molverhältnis von z. B. 4:3 unter Eliminierung von MgCl₂ und unter Bildung von bisher unbekannten Ruthenium- und Iridiumcarbiden der empirischen Zusammensetzung Ru₄C₆(MgCl₂)ₚ(THF)_{q} (**1**) bzw. Ir₄C₆(MgCl₂)ₚ(THF)_{q} (**2**) 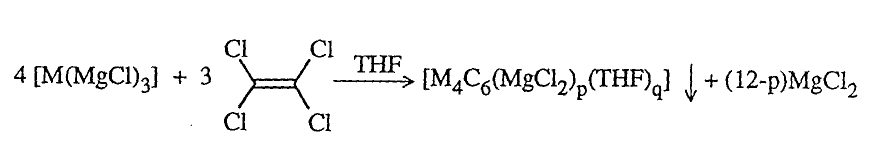 **1**: M = Ru, **2**: M = Ir, p = 0-2, q = 0-2

Die Reaktionen werden bevorzugt bei Temperaturen zwischen 0°C und der Siedetemperatur des verwendeten Lösungsmittels durchgeführt.

Die schwarzen, diamagnetischen in verd. Säuren unlöslichen Feststoffe **1** bzw. **2** sind röntgenamorph; nach dem Tempern (600 °C) erscheinen im Röntgenpulverdiagramm Reflexe von Ru- bzw. Ir-Metall. Eine Untersuchung von **1** bzw. **2** mit Hilfe der matrixunterstützten Laserdesorptions/Ionisations-Massenspektrometrie (MALDI; C.A. Mitchell et. al., *Angew. Chem.* **1996,** *108*, 1076) ergab u.a. Massenpeaks entsprechend RuₓC_{y}, x = 1-8, y = 3-8, und IrₓC_{y}, x = 1-3, y = 2,3 (Messungen von Ch. Yeretzian, Inst. für Physikal. und Theoreth. Chemie der TU München, 1995).

Der Feststoff **1** wurde auch mit Hilfe der EXAFS-Methode (**E**xtended **X**-ray **A**bsorption **F**ine **S**tructure) untersucht und dabei folgendes Ergebnis erhalten:

**Tabelle 1**

| Rückstreuer | Rückstreuerabstand [Å] | Koodinationszahl |
|---|---|---|
| Ru | 2.64 | 2 - 3 |
| C | 2.08 | 2 |

Nach den Ergebnissen des EXAFS-Spektrums, besteht **1** aus extrem kleinen Ru-Clustern (3-4 Ru-Atome), die über direkte Bindungen an C-Atome gebunden sind.

Das darstellbare Ru-Carbid **1** erweist sich als ein hochaktiver heterogener Katalysator für die Hydrierung von als schwierig hydrierbar geltenden Substraten wie Benzol, Toluol und 3-Pentanon, die sich mit **1** als Katalysator bei Raumtemperatur (RT) und Normaldruck quantitativ zu Cyclohexan, Methylcyclohexan und 3-Pentanol (Abb. 1) hydrieren lassen. Bei der Hydrierung von Toluol unter diesen Bedingungen zeigt der Katalysator eine höhere katalytische Aktivität als die zum Vergleich herangezogenen kommerziellen Ru-Katalysatoren (Abb. 2). Eine im Vergleich zu Ru-Schwarz um Größenordnungen höhere katalytische Aktivität wird mit **1** als Katalysator bei der Hydrierung von Pyridin zu Piperidin (99.7 %ig) unter Druck (Abb. 3) festgestellt (vgl. *Tetrahedron: Asymmetry* **1996**, *7,* 313). Unter H₂-Druck ist auch eine schnelle Hydrierung von Toluol zu Methylcyclohexan mit **1** als Katalysator möglich (Abb. 4). Mit **1** als Katalysator läßt sich auch Phenol zu Cyclohexanol hydrieren. Thymol wird mit **1** als Katalysator zu den vier Stereoisomeren von Menthol hydriert.

Weitere Beispiele für Übergangsmetallcarbide dargestellt ausgehend von anorganischen Grignard-Reagenzien und perchlororganischen Verbindungen sind Ru-carbide wie Ru₄C₃(MgCl₂)₀₋₁(THF)₀₋₂ sowie RuC_{2,5}H_{3,6}(MgCl₂)_{0,25} aus Ru(MgCl)₃ und Tetrachlorkohlenstoff bzw. Hexachlorbenzol und Titancarbide wie TiCMg_{0,2}Cl_{0,6}(THF)_{0,25} sowie Ti₂CMg_{0.4}Cl(THF)_{ca. 0,6} aus [Ti(MgCl)₂] (*J. Organometal. Chem.* **1993**, 459, 87) und Tetrachlorethen bzw. Tetrachlorkohlenstoff. Das EXAFS-Spektrum des aus [Ti(MgCl)₂] und Tetrachlorethen erhältlichen Titancarbids weist einen Unterschuß an Kohlenstoff auf (TiC₁₋ₓ) und enthält außerdem noch eine weitere, unbekannte, kohlenstoffreichere Ti-C-Komponente.

Metallcarbide wurden bisher nach folgenden Methoden dargestellt: 1. Durch Zusammenbringen der Metalle oder Metalloxide mit Kohlenstoff bei Temperaturen oberhalb von 1000 °C; 2. Aus Metallen oder Metallverbindungen und einer gasförmigen kohlenstoffhaltigen Verbindung (CO, CH₄); 3. Aus gasförmigen Vorläufern durch Abscheidung aus der Gasphase zum Zwecke der Darstellung von dünnen Carbidschichten; 4. Reduktion von Metallhalogeniden mit LiBEt₃H in einem organischen Lösungsmittel, z.B. THF.

Der technische Fortschritt des vorliegenden Verfahrens liegt darin, daß neue wie auch die bisher bekannten Übergangsmetallcarbide auf einem neuen Syntheseweg, unter extrem milden Reaktionsbedingungen und mit vorteilhaften anwendungstechnischen Eigenschaften (hohe katalytische Aktivität, feinste Verteilung) erzeugt werden können.

Die nachfolgend beschriebenen Versuche wurden in Argonatmosphäre durchgeführt. Es wurden luft- und wasserfreie Lösungsmittel verwendet.

### Beispiel 1

Zu einer Lösung von 10.2 mmol [Ru(MgCl)₃] (dargestellt nach Chem. Mater. 1995, 7, 1153; Mg²⁺-Gehalt: 31.8 mmol, Cl⁻-Gehalt 31.0 mmol) in 50 ml THF wurde tropfenweise und unter Rühren innerhalb einer Stunde eine Lösung von 0.73 ml (7.14 mmol) Tetrachlorethen in 10 ml THF gegeben, wobei sich das Reaktionsgemisch zeitweise bis auf 40 °C erwärmte. Der Ansatz wurde weitere 4 h bei Raumtemperatur gerührt und anschließend 16 h auf Rückfluß gekocht. Nach dem Verdünnen mit 20 ml THF wurde die schwarze Suspension über eine D-4 Glasfritte filtriert, der Feststoff dreimal mit THF gewaschen und anschließend 3 h im Hochvakuum bei 100 °C getrocknet. Erhalten wurden 1.44 g des Ru-Carbids 1 als schwarzes, amorphes Pulver. Elementaranalyse: Ru 62.59 %, C 21.27 %, H 2.23 %, Mg 5.07%, Cl 5.94 %.
Das so erhaltene 1 wurde als Hydrierkatalysator (RuC-Kat; Beispiele 3-9) eingesetzt. Der Cl-Gehalt des Filtrats betrug (nach Volhard) 57 mmol. Der Zuwachs an Cl-Ionen in Lösung (26 mmol) entspricht einem Umsatz von Tetrachlorethen von 91 %. Das restliche Chlor (2.4 mmol) befindet sich lt. EA in 1, so daß die Cl-Bilanz 99.7 % beträgt.

### Beispiel 2

Die Darstellung und Isolierung des Ir-Carbids 2 erfolgte analog der des Ru-Carbids 1. Ausgangsstoffe: 22.5 ml einer [Ir(MgCl)₃]-Lösung in THF mit 3.33 mmol Ir, 10.4 ml Mg²⁺, 10.4 mmol Cl⁻ und eine Lösung von 0.21 ml (2.06 mmol) Tetrachlorethen in 2 ml THF; Zutropfzeit: 0.5 h; maximaler Anstieg der Temperatur: 50 °C. Anschließende Reaktionszeit bei Raumtemperatur: 18 h. Ausbeute des Ir-Carbids 2 als schwarzes, amorphes Pulver: 0.78 g.
Elementaranalyse: Ir 72.18 %, C 13.46 %, H 1.52 %, Mg 3.96 %, Cl 4.08 %. Der Cl-Gehalt des Filtrates: 18,6 mmol; der Zuwachs an Cl⁻-Ionen in Lösung (8.2 mmol) entspricht einem Umsatz von Tetrachlorethen von 99.5 %.

### Beispiel 3 (Anwendungsbeispiel)

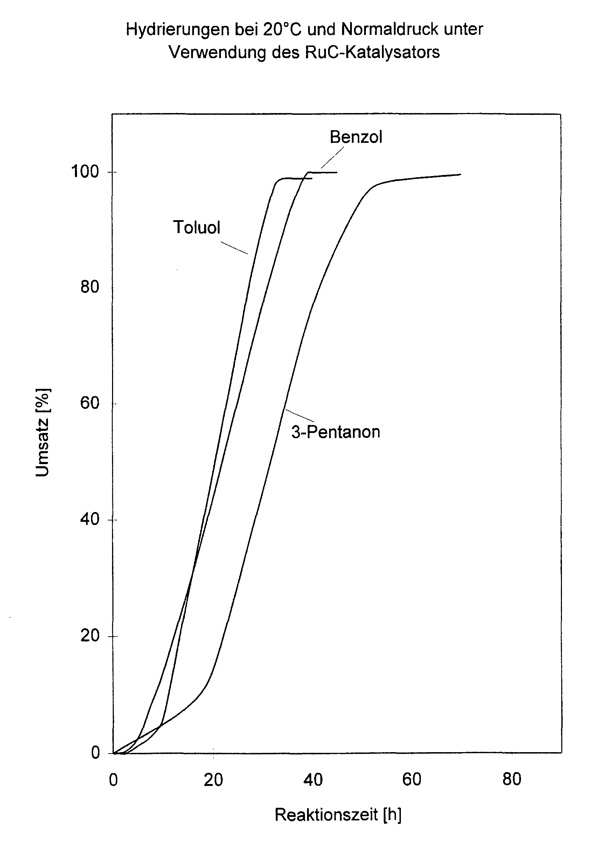

1.1 ml (12.3 mmol) Benzol wurden mit 23.6 mg (1.5 Gew.%) des Ru-Carbid-Katalysators 1 (RuC-Kat.; Beispiel 1) versetzt. Die Suspension wurde bei Raumtemperatur und Normaldruck unter Wasserstoffgas, das aus einer automatisch registrierenden Gasbürette nachgeliefert werden konnte, gesetzt, wobei unter intensiver Rührung die Wasserstoffabsorption begann. Bis zur Beendigung der Hydrierung unter diesen Bedingungen (s. Abb. 1) wurden 862 ml H₂ (21 °C, Normaldruck, 97 % d. Th.) verbraucht. Das durch Filtration vom Katalysator befreite Hydrierprodukt bestand nach GC-Analyse zu 99.97 % aus Cyclohexan..

### Beispiel 4 (Anwendungsbeispiel)

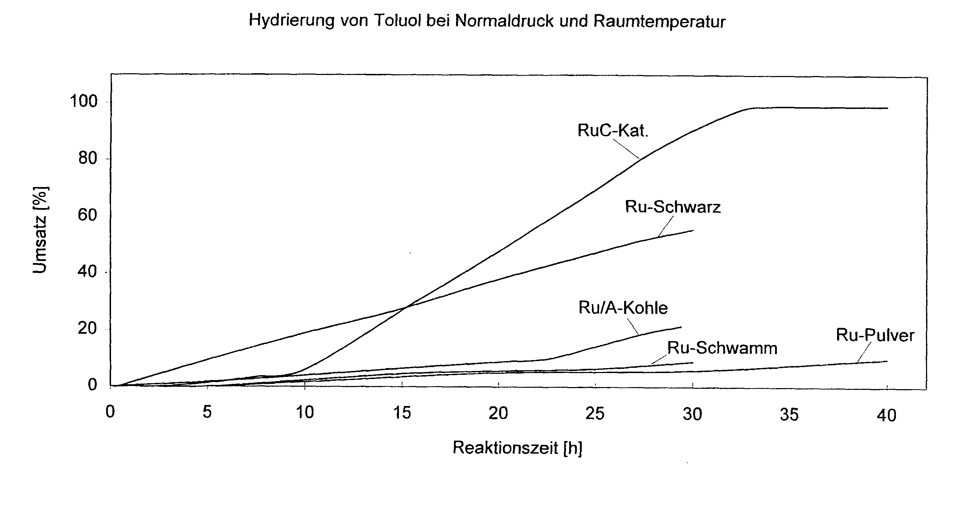

Die Hydrierung von 1.3 ml (12.2 mmol) Toluol in Gegenwart von 24.2 mg des RuC-Katalysators (1.5 Gew.% Ru) wurde analog Beispiel 3 durchgeführt. Wasserstoffaufnahme: 880 ml H₂ von 20 °C, Normaldruck (s. Abb. 1 und 2; 99 % d. Th). Hydrierprodukt: 99 %iges Methylcyclohexan. Wie der Abb. 2 zu entnehmen ist, zeigt der RuC-Katalysator unter den angewandten Hydrierbedingungen eine höhere katalytische Aktivität als Ru-Schwarz, Ru-Schwamm, Ru-Pulver oder Ru auf A-Kohle.

### Beispiel 5 (Anwendungsbeispiel)

Die Hydrierung von 1.0 ml (9.5 mmol) 3-Pentanon in Gegenwart von 23.3 mg des RuC-Katalysators (1.8 Gew.% Ru) wurde analog Beispiel 3 durchgeführt. Wasserstoffaufnahme: 225 ml H₂ von 21 °C, Normaldruck (s. Abb. 1; 99 % d. Th). Hydrierprodukt: 99.6 %iges 3-Pentanol

### Beispiel 6 (Anwendungsbeispiel)

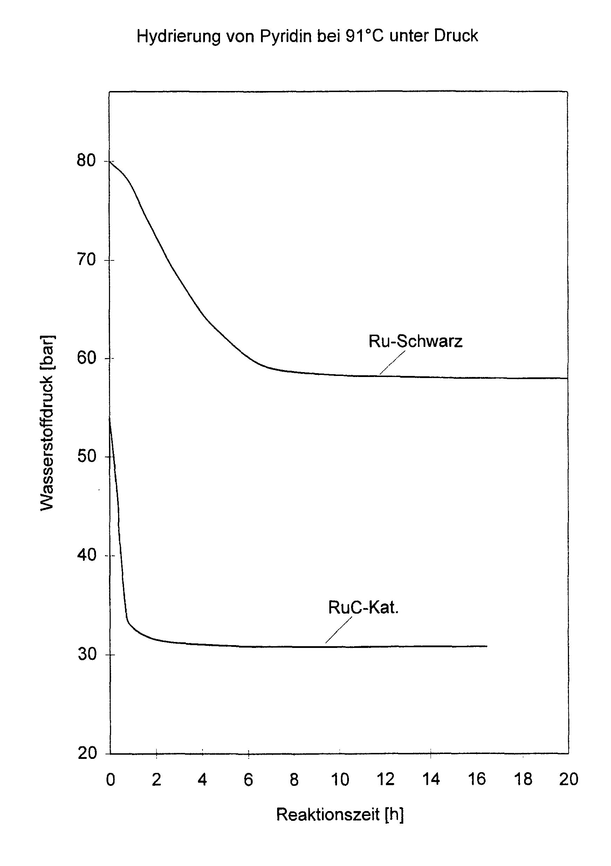

Die Hydrierung von 5.0 ml (61.8 mmol) abs. Pyridin in Gegenwart von 39.3 mg des RuC-Katalysators (0.5 Gew.% Ru) wurde in einem mit einem Magnetrührkern ausgerüsteten Autoklaven bei 91 °C (Innentemperatur) und einem Wasserstoffanfangsdruck von 54 bar durchgeführt. Den Verlauf der Hydrierreaktion im Vergleich zu der entsprechenden Hydrierung von Pyridin mit Ru-Schwarz als Katalysator zeigt die Abb. 3. Da sich der RuC-Katalysator während der Hydrierung scheinbar auflöste, wurde das Hydrierprodukt durch Destillation im Vakuum (0.1 mbar) vom Katalysator abgetrennt. Hydrierprodukt: 5.10 g (97 % d. Th.) Piperidin von 99,7 %iger Reinheit.

### Beispiel 7 (Anwendungsbeispiel)

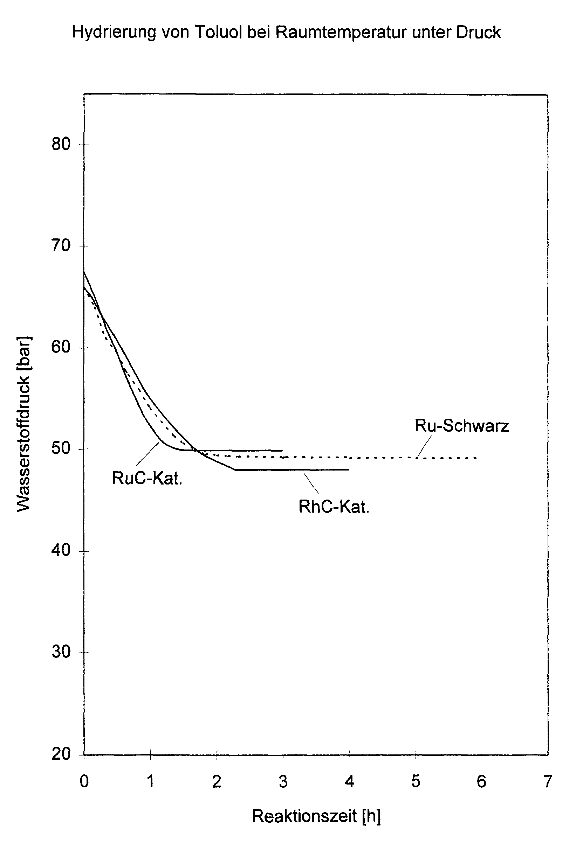

Die Hydrierung von 5.0 ml (47 mmol) Toluol in Gegenwart von 36.2 mg des RuC-Katalysators (0.5 Gew.% Ru) wurde unter H₂-Druck von 54 bar (Anfangsdruck) und Raumtemperatur analog Beispiel 6 durchgeführt. Den Verlauf der H₂-Aufnahme zeigt Abb. 4. Das durch Filtration vom Katalysator getrennte Hydrierprodukt (4.53 g) bestand nach GC-Analyse zu 99.3 % aus Methylcyclohexan.

### Beispiel 8 (Anwendungsbeispiel)

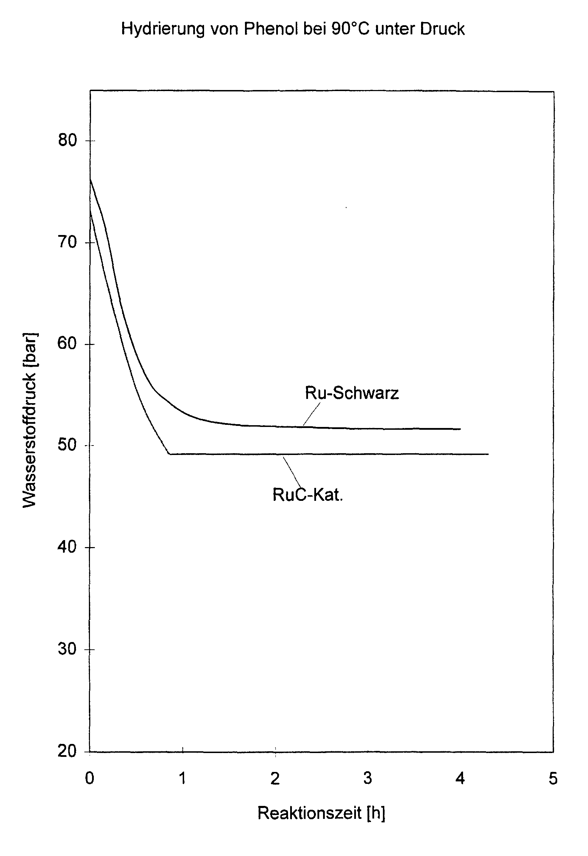

Die Hydrierung von 5.01 g (53.2 mmol) Phenol in 10 ml THF in Gegenwart von 42 mg des RuC-Katalysators (0.5 Gew.% Ru) wurde unter H₂-Anfangsdruck von 73 bar und 90 °C analog Beispiel 6 durchgeführt. Den Verlauf der H₂-Aufnahme zeigt die Abb. 5. Das durch Filtration vom Katalysator getrennte Hydrierprodukt (13.1 g) enthielt laut GC 39,4 % Cyclohexanol (Rest THF) und keine weiteren Bestandteile.

### Beispiel 9 (Anwendungsbeispiel)

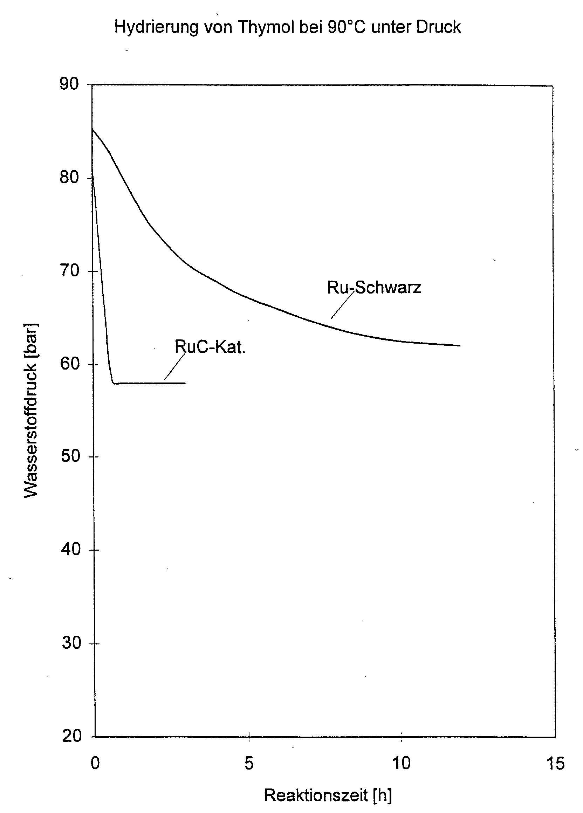

Die Hydrierung von 9.74 g (64.8 mmol) Thymol in 10 ml THF in Gegenwart von 81 mg des RuC-Katalysators (0.5 Gew.% Ru) wurde unter H₂-Anfangsdruck von 81 bar und 90 °C analog Beispiel 6 durchgeführt. Den Verlauf der H₂-Aufnahme: zeigt die Abb. 6. Das durch Filtration vom Katalysator getrennte Hydrierprodukt (17.3 g) bestand zu 55.8 % (Rest THF) nur aus den vier Stereoisomeren von Menthol. Isomerenverteilung: Neomenthol 10.0 %, Neoisomenthol 50.2 %, Menthol 6.4 %, Isomenthol 33.4 %.

### Beispiel 10 (Anwendungsbeispiel)

Die Hydrierung von 9.70 g (64.6 mmol) Thymol in 10 ml THF in Gegenwart von 68.5 mg des IrC-Katalysators (Beispiel 2, 0.5 Gew.% Ir) wurde unter H₂-Anfangsdruck von 80 bar und 90 °C analog Beispiel 6 durchgeführt. Das durch Filtration vom Katalysator getrennte Hydrierprodukt bestand (abgesehen vom THF) zu 16.3 % aus Thymol und zu 83.7 % aus den vier Stereoisomeren von Menthol. Isomerenverteilung: Neomenthol 23.4 %, Neoisomenthol 68.4 %, Menthol 5.5 %, Isomenthol 2.7 %.

### Beispiel 11

Die Darstellung eines Ru-Carbids aus [Ru(MgCl)₃] und CCl₄ erfolgte analog der des Ru-Carbids 1. Ausgangsstoffe: 20.0 ml einer [Ru(MgCl)₃]-Lösung in THF mit 3.8 mmol Ru und 11.9 mmol Cl⁻ und eine Lösung von 0.24 ml (2.49 mmol CCl₄ in 4 ml THF. Zutropfzeit: 0.5 h; maximaler Temperaturanstieg: 45 °C. Anschließende Reaktionszeit: 3 h bei Raumtemperatur und 8 h bei Rückflußtemperatur. Ausbeute des Ru-Carbids: 0.46 g (Ru 69.16 %, C 17.53 %, H 2.04 %, Mg 3.36 %, Cl 9.83 %, entsprechend Ru₄C₃(MgCl₂)_{0.8}(THF)_{1.4}. Der Cl⁻-Gehalt des Filtrates: 20.3 mmol; der Zuwachs an Cl⁻-Ionen in Lösung (8.4 mmol) entspricht einem Umsatz von Tetrachlorkohlenstoff von 84 %.

### Beispiel 12

Die Darstellung eines Ru-Carbids aus [Ru(MgCl)₃] und Hexachlorbenzol erfolgte analog der des Ru-Carbids 1. Ausgangsstoffe: 20.0 ml einer [Ru(MgCl)₃]-Lösung in THF mit 3.8 mmol Ru und 11.9 mmol Cl und eine Suspension von 0.48 g (1.68 mmol C₆Cl₆ in 6 ml THF. Zutropfzeit: 0.5 h; maximaler Temperaturanstieg: 35 °C. Anschließende Reaktionszeit wie im Beispiel 11. Ausbeute des Ru-Carbids: 0.49 g (Ru 64.78 %, C 19.01 %, H 2.35 %, Mg 3.56 %, Cl 11.69 %. Der Cl⁻-Gehalt des Filtrates: 19.8 mmol; der Zuwachs an Cl⁻-Ionen in Lösung (7.9 mmol) entspricht einem Umsatz von C₆Cl₆ von 78 %.

### Beispiel 13

Die Darstellung eines Ti-Carbids aus [Ti(MgCl)₂ ·0.5 MgCl₂] (J. Organometal. Chem. 1993, 459, 87) und Tetrachlorethen erfolgte analog der des Ru-Carbids 1. Ausgangsstoffe: 200 ml einer [Ti(MgCl)₂ 0.5 MgCl₂]-Lösung in THF mit 18.7 mmol Ti und 58.4 mmol Cl⁻ und 0.84 ml (8.22 mmol Tetrachlorethen. Zutropfzeit: 0.5 h; maximaler Temperaturanstieg: 40 °C. Anschließende Reaktionszeit: 2 h bei Raumtemperatur und 17 h bei Rückflußtemperatur. Ausbeute des Ti-Carbids: 2.02 g (Ti 42.75 %, C 21.42 %, H 2.34 %, Mg 4.75 %, Cl 18.95 %, entsprechend TiCMg_{0.2}Cl_{0.6}(THF)_{0.25}. Der Cl⁻-Gehalt des Filtrates: 88.2 mol; der Zuwachs an Cl -Ionen in Lösung (29.8 mmol) entspricht einem Umsatz von Tetrachlorethen von 91 %.

### Beispiel 14

Die Darstellung eines Ti-Carbids aus [Ti(MgCl)₂ ·0.5 MgCl₂] (s. Beispiel 13) und CCl₄ erfolgte analog der des Ru-Carbids 1. Ausgangsstoffe: 120 ml einer [Ti(MgCl)₂ 0.5 MgCl₂]-Lösung in THF mit 11.2 mmol Ti und 35.0 mmol Cl und 0.50 ml (5.2 mmol) CCl₄. Zutropfzeit: 0.5 h; maximaler Temperaturanstieg: 42 °C. Anschließende Reaktionszeit: Siehe Beispiel 13. Ausbeute des Ti-Carbids: 0.9 g (Ti 43.34 %, C 19.01 %, H 4.05 %, Mg 4.70 %, Cl 16.58 %, entsprechend Ti₂CMg_{0.4}Cl_{1.0}(THF)_{ca.0.6}. Der Cl⁻-Gehalt des Filtrates: 50.0 mmol; der Zuwachs an Cl -Ionen in Lösung (15 mmol) entspricht einem Umsatz von CCl₄ von 72 %.

### Beispiel 15

Die Darstellung und Isolierung eines Rh-Carbids erfolgte analog der des Ru-Carbids 1 Beispiel 1. Ausgangsstoffe: 30 ml einer [RhMgCl MgCl₂]-Lösung in THF mit 2.94 mmol Rh und 8.82 mmol Cl⁻ und 0.067 ml (0.66 mmol Tetrachlorethen. Reaktionszeit: 24 h bei Raumtemperatur. Ausbeute des Rh-Carbids als schwarzes amorphes Pulver: 0.39 g. Der Cl⁻-Gehalt des Filtrates: 10.9 mmol; der Zuwachs an Cl⁻-Ionen in Lösung (2.13 mmol) entspricht einem Umsatz von Tetrachlorethen von 81 %.

### Beispiel 16 (Anwendungsbeispiel)

Die Hydrierung von 5.0 ml (47 mmol) Toluol in Gegenwart von 33.7 mg des RhC-Katalysators (Beispiel 15; 0.5 Gew.% Rh) wurde unter H₂ -Anfangsdruck von 54 bar bei Raumtemperatur analog Beispiel 6 durchgeführt (Abb. 4). Das durch Filtration vom Katalysator getrennte Hydrierprodukt (4.47 g) bestand zu 100 % aus Methylcyclohexan.

## Patentansprüche

1. Verfahren zur Herstellung von Übergangsmetallcarbiden **dadurch gekennzeichnet, daß** man die aus Übergangsmetallchloriden und Magnesium bzw. magnesiumorganischen Verbindungen in etherischen Lösungsmitteln erhältlichen, löslichen Komplexe [M (MgCl)ₘ(MgCl₂)ₙ], M = Übergangsmetall der Gruppen 4 - 10 des Periodensystems, m = 1,2,3, n = 0 - 1, mit perchlororganischen Verbindungen umsetzt.

2. Verfahren nach Anspruch 1 **dadurch gekennzeichnet, daß** Tetrachlorethen, Tetrachlorkohlenstoff und Hexachlorbenzol als perchlororganische Verbindungen verwendet werden.

3. Verfahren nach Anspruch 1 und 2 **dadurch gekennzeichnet, daß** die Reaktion bevorzugt in einem etherischen Lösungsmittel durchgeführt wird

4. Verfahren nach Anspruch 1-3 **dadurch gekennzeichnet, daß** als Lösungsmittel Tetrahydrofuran verwendet wird.

5. Verfahren nach Anspruch 1-4 **dadurch gekennzeichnet, daß** die Reaktion bei Temperaturen von 0 °C bis zur Siedetemperatur des betreffenden Lösungsmittels durchgeführt wird.

6. Ru-Carbid Ru₄C₆(MgCl₂)ₚ(THF)_{q}, p = 0 - 2, q = 0 - 2, hergestellt aus [Ru(MgCl)₃] und Tetrachlorethen.

7. Ir-Carbid, Ir₄C₆(MgCl₂)ₚ(THF)_{q}, p = 0 - 2, q = 0 - 2, hergestellt aus [Ir(MgCl)₃] und Tetrachlorethen.

8. Ru-Carbid Ru₄C₃(MgCl₂)₀₋₁(THF)₀₋₂, hergestellt aus [Ru(MgCl)₃] und Tetrachlorkohlenstoff.

9. Ru-Carbid RuC_{2.5}H_{3.6}(MgCl₂)_{0.25},hergestellt aus [Ru(MgCl)₃] und Hexachlorbenzol.

10. Titancarbid TiCMg_{0.2}Cl_{0.6} (THF)_{0.25}, hergestellt aus [Ti(MgCl)₂] und Tetrachlorethen.

11. Titancarbid Ti₂CMg_{0.4}Cl_{1.0}(THF)_{ca.0.6}, hergestellt aus [Ti(MgCl)₂] und Tetrachlorkohlenstoff.

12. Verwendung der Metallcarbide gemäß Ansprüchen 6-11 und der gemäß Ansprüchen 1-5 herstellbaren Metallcarbide als heterogene Hydrierkatalysatoren.

13. Verwendung der Metallcarbide gemäß Ansprüchen 6-11 und der gemäß Ansprüchen 1-5 herstellbaren Metallcarbide als Hydrierkatalysatoren für Benzol, Toluol, Phenol oder Thymol oder Gemischen dieser Ausgangsprodukte.

## Claims

1. A process for the preparation of transition metal carbides, **characterized in that** the soluble complexes [M(MgCl)ₘ(MgCl₂)ₙ], M = transition metal of groups 4 to 10 of the Periodic Table, m = 1, 2, 3, n = 0-1, obtainable from transition metal chlorides and magnesium or organomagnesium compounds in ether solvents, are reacted with perchloroorganic compounds.

2. The process according to claim 1, **characterized in that** tetrachloroethene, carbon tetrachloride or hexachlorobenzene is used as said perchloroorganic compound.

3. The process according to claims 1 and 2, **characterized in that** the reaction is preferably performed in an ether solvent.

4. The process according to claims 1 to 3, **characterized in that** tetrahydrofuran is used as said solvent.

5. The process according to claims 1 to 4, **characterized in that** the reaction is performed at temperatures from 0 °C to the boiling temperature of the solvent employed.

6. The Ru carbide Ru₄C₆(MgCl₂)ₚ(THF)_{q}, p = 0-2, q = 0-2, prepared from [Ru(MgCl)₃] and tetrachloroethene.

7. The Ir carbide Ir₄C₆(MgCl₂)ₚ(THF)_{q}, p = 0-2, q = 0-2, prepared from [Ir(MgCl)₃] and tetrachloroethene.

8. The Ru carbide Ru₄C₃(MgCl₂)₀₋₁(THF)₀₋₂, prepared from [Ru(MgCl)₃] and carbon tetrachloride.

9. The Ru carbide Ru₄C_{2.5}H_{3.6}(MgCl₂)_{0.25}, prepared from [Ru(MgCl)₃] and hexachlorobenzene.

10. The titanium carbide TiCMg_{0.2}Cl_{0.6}(THF)_{0.25}, prepared from [Ti(MgCl)₂] and tetrachloroethene.

11. The titanium carbide Ti₂CMg_{0.4}Cl_{1.0}(THF)ca._{0.6}, prepared from [Ti(MgCl)₂] and carbon tetrachloride.

12. Use of the metal carbides according to claims 6 to 11 and of the metal carbides preparable according to claims 1 to 5 as heterogeneous hydrogenation catalysts.

13. Use of the metal carbides according to claims 6 to 11 and of the metal carbides preparable according to claims 1 to 5 as hydrogenation catalysts for benzene, toluene, phenol or thymol or mixtures of these starting materials.

## Revendications

1. Procédé de fabrication de carbures de métaux de transition, **caractérisé en ce que** l'on fait réagir les complexes solubles [M (MgCl)ₘ(MgCl₂)ₙ], où M = métal de transition des groupes 4 à 10 de la classification périodique des éléments, m = 1, 2, 3, n = 0-1, pouvant être obtenus à partir de chlorures de métaux de transition et de magnésium ou, selon les cas, de composés organomagnésiens dans des solvants éthérés, avec des composés organoperchlorés.

2. Procédé selon la revendication 1, **caractérisé en ce que** l'on utilise en tant que composés organoperchlorés du tétrachloréthène, du tétrachlorocarbone et de l'hexachlorobenzène.

3. Procédé selon la revendication 1 et 2, **caractérisé en ce que** la réaction est de préférence conduite dans un solvant éthéré.

4. Procédé selon la revendication 1 à 3, **caractérisé en ce que** l'on utilise comme solvant le tétrahydrofurane.

5. Procédé selon la revendication 1 à 4, **caractérisé en ce que** la réaction est conduite à des températures comprises entre 0 °C et la température d'ébullition du solvant concerné.

6. Carbure de Ru Ru₄C₆(MgCl₂)ₚ(THF)_{q}, où p = 0-2, q = 0-2, fabriqué à partir de [Ru(MgCl)₃] et de tétrachloréthène.

7. Carbure d'Ir Ir₄C₆(MgCl₂)ₚ(THF)_{q}, où p = 0-2, q = 0-2, fabriqué à partir de [Ir(MgCl)₃] et de tétrachloréthène.

8. Carbure de Ru Ru₄C₃(MgCl₂)₀₋₁(THF)₀₋₂, fabriqué à partir de [Ru(MgCl)₃] et de tétrachlorocarbone.

9. Carbure de Ru RuC_{2,5}H_{3,6}(MgCl₂)_{0,25}, fabriqué à partir de [Ru(MgCl)₃] et d'hexachlorobenzène.

10. Carbure de titane TiCMg_{0,2}Cl_{0,6}(THF)_{0,25}, fabriqué à partir de [Ti(MgCl)₂] et de tétrachloréthène.

11. Carbure de titane Ti₂CMg_{0,4}Cl_{1,0}(THF)_{env. 0,6}, fabriqué à partir de [Ti(MgCl)₂] et de tétrachlorocarbone.

12. Utilisation des carbures métalliques selon les revendications 6 à 11 et des carbures métalliques pouvant être fabriqués selon les revendications 1 à 5 en tant que catalyseurs d'hydrogénation hétérogènes.

13. Utilisation des carbures métalliques selon les revendications 6 à 11 et des carbures métalliques pouvant être fabriqués selon les revendications 1 à 5 en tant que catalyseurs d'hydrogénation du benzène, du toluène, du phénol ou du thymol, ou de mélanges de ces produits de départ.
